# EUROPEAN PATENT SPECIFICATION

(11) **EP 2 163 265 B1**
(45) Date of publication and mention of the grant of the patent: **29.08.2012**
(21) Application number: 08167672.8
(22) Date of filing: 27.10.2008
(51) Int. Cl.: A61L 9/22, B01D 53/32

(54) **Method and device for removal of malodorous compounds during asphalt production**
Verfahren und Vorrichtung zur Entfernung übelriechender Stoffe aus der Asphaltherstellung
Méthode et dispositif pour l'élimination des composés malodorants au cours de la production d'asphalte

(30) Priority: 09.09.2008 PL 38605508
(43) Date of publication of application: 17.03.2010
(73) Proprietor: Politechnika Lubelska, 20-219 Lublin (PL)
(72) Inventor: Ozonek, Janusz, 20-470 Lublin (PL); Pawlowski, Lucjan, 20-027 Lublin (PL); Tyszko, Wlodzimierz, 03-833 Warsaw (PL); Czech, Andrzej, 81-759 Sopot (PL); Piotrowicz, Adam, 20-858 Lublin (PL)
(74) Representative: Kaminski, Piotr

(56) References cited:
- EP-A- 2 025 351
- DE-A1- 19 903 022
- US-A- 5 458 748
- US-A1- 2006 182 672
- US-A1- 2007 196 254

## Description

The invention concerns a method and device for removal of malodorous compounds in the process of asphalt production and a device to be used to this effect.

In asphalt-producing plants, where a mixture of asphaltic concrete with filling and improving additives is prepared, large volumes of gases that are contaminated with malodorous compounds are formed and released to the atmosphere. These are mainly organic high-molecular compounds of chain or polycyclic structures. They are often carcinogenic. Because of these emissions the plants are very oppressive for the environment and its inhabitants. It is the reason why attempts are made to reduce or eliminate malodorous compounds from the asphalt production process.

US2007196254 relates to a method for treating fumes generated during the production, conversion and/or handling of oil-based products and a device for carrying out said method. The invention further relates to the use of the method or device in which the trapping device comprises at least one fluidised bed of granular material in the preparation of a granular material for use in production of road materials.

EP2025351, prior art for the assessment of novelty only, air purifier comprises a blower, an ozone generator, an ozone neutralizer, an air duct an air inlet opening and an air outlet opening. The ozone generator and the ozone neutralizer are located between the air inlet opening and the air outlet opening, in the air duct. They are exposed to airflow from the blower. The ozone generator is located at the air inlet- or outlet opening. A microfilter is placed between the ozone generator and the ozone neutralizer.

The known methods for the removal and/or decomposition of malodorous compounds during asphalt production are realized in separate installations that are rather expensive because of their dimensions, energy consumption and efficiency. Widely used methods for malodorous compound removal mainly involve the application of absorption or adsorption, thermal or catalytic combustion, chemical oxidation or dissipation in the atmosphere.

American patent No 527 1767 describes a method which involves addition of plant oils during asphalt production process. This proves to reduce malodorous compound emission or change their odour.

The American patent specification No US 6451252 presents a method for malodorous compounds removal, mainly of ammonia and hydrogen disulfide, with the application of a plasma-chemical reactor with simultaneous ozone addition to the purified gases.

The Korean patent application No KR 2001-74564 describes a method and an apparatus for the removal of volatile organic compounds and offensive odours - with the use of non-equilibrium plasma, where volatile organic compounds and offensive odour-contaminated gases are directed to a chamber where non-thermal plasma is generated by high voltage of elevated frequency. This induces reactions between volatile organic compounds and offensive odours contained in the gases which causes their partial reduction and oxidation. volatile organic compounds and offensive odours contained in the gases which causes their partial reduction and oxidation.

The disadvantage of the above-mentioned methods is that they can be applied only to decompose inorganic compounds (e.g. ammonia, hydrogen sulphide) or volatile inorganic compounds.

The invention is defined by claims 1 and 2.

The essence of the present method for removal of malodorous compounds during asphalt production in the low-temperature plasma is that the gases, after prior purification from dust and small solid particles are send by use of fan to a low-temperature plasma generator, where they get decomposed.

The device to perform the above-described process of malodorous compound removal is equipped with a plasma generator and mesh electrodes and it is composed of a cylindrical dielectric casing that is centred by dielectric rings. Inside the casing there is a high-voltage mesh electrode and an earthed mesh electrode, both are cylinder-shaped and have the same length and mesh size and they are located inside and outside of the cylindrical dielectric, preferably of small thickness. To generate discharges, high voltage, preferably of elevated frequency, is applied via a wire to the high-voltage mesh electrode The mesh electrode is grounded by a wire.

An advantageous effect of the described invention is that it decomposes high-molecule malodorous compounds that are produced during asphalt production and thereby makes them harmless for humans and the environment..

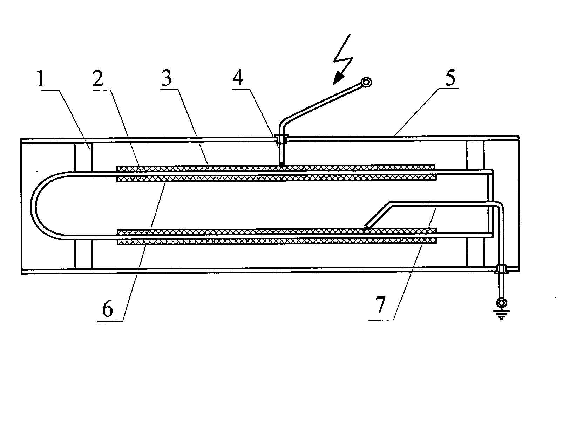

An embodiment of the device according to present invention is presented in the drawing in longitudinal section view.

Malodorous compounds that are formed during asphalt production are first cleaned out of dust and small solid particles and then directed by a fan to a low-temperature plasma generator, where they get decomposed and/or changed into less odorous or non-odorous simple compounds, which results from various reaction mechanisms that occur in the non-thermal plasma environment.

The described device for removing malodorous compounds distinguishes itself by including an element for generating plasma environment that consist of cylindrical mesh electrodes 3 and 6, and electrode 3 is a high-voltage electrode and electrode 6 is an earthed electrode. The electrodes are situated inside and outside of a cylinder-shaped dielectric 2. High voltage, preferably of elevated frequency, is applied via the wire 4 to the high-voltage mesh electrode 3 in order to generate electrical discharges, while the earthed electrode 6 is grounded by the wire 7. All the elements are placed in a dielectric casing 5 which is centred by dielectric rings 1. Example Gaseous mixture containing malodorous compounds formed during asphalt production, following removal of dust and small solid particles, is directed by a fan to the discharge zone of a plasma generator at the flow rate of 10 m3/h. Electrical current at the 11kV voltage and 50Hz frequency has been applied to the electrodes 3 and 6, isolated from each other by a 2mm-thick cylinder-shaped glass dielectric layer 2. Degradation of malodorous compounds occurs on the mesh electrode surface 3 and in its immediate environment. Degree of malodorous compound decomposition has been determined for gas leaving the plasma generator.

For example, more than 90% of dicyclopentane contained in malodorous compounds formed during asphalt production has been decomposed and for dichlorobenzenes and trichlorobenzenes the degree of decomposition exceeded 70%.

At the density of specific energy that is supplied to the reactor of about 4 Wh/m3, the efficiency of malodorous compound removal exceeds 80%.

## Claims

1. A device for removal of malodorous compound which are formed during process of asphalt production, equipped with a plasma generator and mesh electrodes, **characterised in that** it is composed of a cylindrical dielectric casing (5) centred by dielectric rings (1) and in the casing there is a high-voltage mesh electrode (3) and un earthed mesh electrode (6) and both electrodes (3 6) are cylinder-shaped and have the same length and mesh size and they are placed inside and outside of a cylindrical dielectric (2).

2. The device according to claim 2, **characterised in that** the high-voltage mesh electrode (3) is supplied via a wire (4) with high voltage, preferably of elevated frequency, to generate electrical discharges, while the earthed mesh electrode (6) is grounded by a wire (7).

3. A method for removal of malodorous compounds which are formed during process of asphalt production using the device according to claim 1 or 2, the method comprising sending the gases after prior purification from dust and small solid particles by a fan to said device, where their decomposition takes place.

## Patentansprüche

1. Vorrichtung zum Entfernen von beim Asphaltherstellungsprozess anfallenden übelriechenden Verbindungen, die mit einem Plasmagenerator und Netzelektroden ausgestattet ist, **dadurch gekennzeichnet dass** sie aus einem zylindrischen dielektrischen Gehäuse (5) besteht das mit dielektrischen Ringen (1) zentriert ist, und sich im Gehäuse eine Hochspannungsnetzelektrode (3) und eine geerdete Netzelektrode (6) befinden, wobei die beiden Elektroden (3, 6) zylinderförmig sind und dieselbe Länge und Maschenweite aufweisen und innerhalb und außerhalb eines zylindrischen Dielektrikums (2) angeordnet sind.

2. Vorrichtung nach Anspruch 2, **dadurch gekennzeichnet, dass** die Hochspannungsnetzelektrode (3) mittels einer Leitung (4) mit Hochspannung vorzugsweise mit einer erhöhten Frequenz, versorgt wird, um elektrische Entladungen hervorzurufen, wobei die geerdete Netzelektrode (6) mittels einer Leitung (7) geerdet ist.

3. Verfahren zum Entfernen von beim Asphaltherstellungsprozess anfallenden übelriechenden Verbindungen unter Verwendung der Vorrichtung nach Anspruch 1 oder 2, wobei das Verfahren das Zuleiten der Gase nach einer vorherigen Reinigung von Staub und kleinen Feststoffpartikeln mittels eines Ventilators zur genannten Vorrichtung umfasst, wo es zu ihrer Zersetzung kommt.

## Revendications

1. Dispositif pour éliminer les composés malodorants formés au cours du procédé de production d'asphalte, comprenant un générateur de plasma et des électrodes à grille, **caractérisé en ce qu'**il se compose d'un boîtier cylindrique et diélectrique (5), centré par des bagues diélectriques (1), tandis que dans un boîtier se place une électrode à grille à haute tension (3) et une électrode à grille de mise à la terre (6), le deux électrodes (3 6) étant en forme de cylindre et comprenant la même longueur et la même taille de maille, et étant placées à l'intérieur et à l'extérieur du diélectrique cylindrique (2)

2. Dispositif selon la revendication 2, **caractérisé en ce que** la haute tension arrive à l'électrode à grille à haute tension (3) par un câble (4) de préférence à fréquence élevée, pour provoquer des décharges électriques, l'électrode à grille de mise à la terre (6) étant mise à la terre par un câble (7)

3. Procédé pour éliminer des composés malodorants formés au cours du procédé de production d'asphalte utilisant le Dispositif selon la revendication 1 ou 2, le procédé comprenant la distribution, par un ventilateur des gaz préalablement purifiés de poussière et de particules solides fines audit dispositif, où ils se décomposent.
